# EUROPEAN PATENT APPLICATION

(11) **EP 3 542 857 A1**
(43) Date of publication of application: **25.09.2019**
(21) Application number: 18168624.7
(22) Date of filing: 20.04.2018
(51) Int. Cl.: A61N 5/06

(54) **BALANCED CONE EXCITATION FOR CONTROLLING REFRACTIVE ERROR AND OCULAR GROWTH TO INHIBIT DEVELOPMENT OF MYOPIA**

(30) Priority: 19.03.2018 US 201862644622 P
(71) Applicant: Rucker, Frances Joan, Boston, MA 02114 (US)
(72) Inventor: Rucker, Francis, Boston, MA 02114 (US); Yoon, Hannah, Boston, ME 02115 (US); Taylor, Christopher Patrick, Quincy, MA 02169 (US)
(74) Representative: Barker Brettell LLP

(57) **Abstract**

Apparatus (66) to provide light stimulation to photoreceptors of an eye of a user in a manner that is suitable for slowing myopia development in an indoor environment wherein the apparatus is arranged to emit light stimulation having a red, R, cone /blue, B, cone excitation ratio in a range of 1.38 and 0.05.

## Description

### TECHNICAL FIELD

Aspects of the present disclosure are generally related to the fields of optometry and ophthalmology, and more particularly to procedures based on relative stimulation of the retinal cones that inhibit development of myopia.

### BACKGROUND

Myopia is a refractive error of the eye. The condition is characterized by the eyeball having an axial length that is mismatched with the focusing power of the cornea and lens such that light rays focus at a point in front of the retina, rather than directly on the surface of the retina. Development of myopia typically begins in childhood and stabilizes in early adulthood.

At birth the human eye is often too short for its optics, a condition which is called hyperopia. A hyperopic eye focuses the image of a distant object behind the retina. With experience and development, the human eye emmetropizes and infantile hyperopia trends towards emmetropia (Mutti et al., 2005).

Longitudinal chromatic aberration (LCA) is an aberration that causes short- and long-wavelength light to be refracted by different amounts, creating wavelength defocus. Wavelength defocus, even in an emmetropic eye, causes shorter wavelengths to be focused in front of the retina, while longer wavelengths are focused behind the retina. Depending on the relative defocus of blue versus red light, the color contrast of the retinal image will change. If the eye is too short for its optics then blue wavelengths will be more in focus than red, whereas if the eye is too long for its optics, red will be more focused than blue. LCA and its interaction with eye length provides a cue in the retinal image that could be used by the emmetropization process to signal whether the eye is too long or too short for its optics (Rucker and Wallman, 2009; Rucker and Wallman, 2012; Rucker, 2013).

There is a correlation between time spent outdoors during childhood and a reduced incidence of myopia (Deng et al., 2010; Dirani et al., 2009; Jones et al., 2007; Onal et al., 2007). The decrease in myopia prevalence was not related to the amount of near work (Rose et al., 2008), nor to the levels of physical activity engaged in while outdoors (Guggenheim et al., 2012). Thus, the evidence suggests that being exposed to natural light is important for the emmetropization process.

Natural light is composed of a broad-spectrum of wavelengths, whereas indoor illumination contains a greater proportion of long- relative to short-wavelengths (i.e., more red than blue). To test the hypothesis that the difference in light spectrum is a factor in the development of myopia, Rucker et al. (2015) used slow-frequency flicker stimuli that mimicked natural light and indoor lighting. The presence of a blue light component in the natural light stimulus reduced the amount of eye growth and myopia that developed.

### SUMMARY

According to the invention there is provided apparatus to provide light stimulation to photoreceptors of an eye of a user in a manner that is suitable for slowing myopia development in an indoor environment wherein the apparatus is arranged to emit light stimulation having a red cone /blue cone excitation ratio in a range of 1.38 and 0.05.

The invention may be viewed as suitable for treating, preventing, or reducing myopia, or the risk thereof, in an indoor environment.

### Temporal Characteristics

The stimulation may be provided in a steady or temporal manner at a frequency of /at least/more than 0 Hz, to 100 Hz (Gottlieb & Wallman 1987). The frequency of light stimulation may be in the range 0.2Hz to 5Hz.

Amplitude of the modulation creates contrast in the red (long-wavelength sensitive (L-)), green (middle-wavelength sensitive (M-)) or blue (short-wavelength sensitive (S-)) cones in a range of 0 to 100%, when calculate using Michelson Contrast (LMSmax-LMSmin)/(LMSmax+LMSmin). Cone contrast can be considered for each cone type or combined to give total contrast of the stimulus.

The amplitude and phase of cone contrast can be useful in producing a therapeutic effect. Experimental results show that with 0.2 Hz sinusoidal, in-phase, modulation, therapeutic effects are found in a narrow range between 28 and 30% blue cone contrast when red cone contrast is 11% to 0%. Modulation in counter-phase reverses the required cone contrast ratios (28-30% red cone contrast, with 0-11% blue cone contrast). Indeed, sinusoidal modulation at 10 Hz **promotes** myopia development with this narrow range of conditions. At 10 Hz, all combinations outside of this narrow range (up to 74 % blue cone contrast and 31% red, or vice versa with counterphase modulation) provides a protective effect.

The light stimulation may be in the form of one or more of the following: flickering light, an image (e.g. a fixed or changing/moving image), a pattern (e.g. a fixed or changing/moving pattern), visual noise/visual distortions (e.g. white noise - a random pattern with a constant power spectral density, pink noise - having equal energy per octave, and so having more low-frequency components than white noise, black noise - a frequency spectrum of predominantly zero power level over all frequencies except for a few narrow bands or spikes, noisy white or black - a non-uniformity in the white or black area of the image, brown noise - random changes in an image, or any type of visual noise.)

The stimulation may comprise sine wave stimulation and/or square wave stimulation.

One some or all of the RGB components of the lighting stimulation may be modulated randomly or in a pre-determined manner with the temporal/spatial characteristics described above.

### Lighting Characteristics

As will be appreciated by those skilled in the art, cone cells, or cones, are one of three types of photoreceptor cells in the retina of mammalian eyes (e.g. the human eye). They are responsible for color vision and function

Blue light included in the light stimulation is at a wavelength of 400-500 nm.

Red light included in the light stimulation is at a wavelength of 600-700nm

Green light included in the light stimulation is at a wavelength of 500-600 nm

The intensity of these different lighting components are manipulated to provide the desired cone excitation in the red, green and blue cones.

Regarding luminosity, the blue light component may be greater than 0.5 lux, 200lux, 300lux, 400lux, 500lux or 600 lux. The luminosity of the combined components of the light may be equal to substantially 680 lux, or may be equal to
substantially 985 lux or as great as 1500 lux.

A light source may be arranged to provide the light stimulation, this may be at a particular luminosity or at a time-varying luminosity.

### Presentation Characteristics

The apparatus may comprise, or be part of, any of the following entities, or is at least in the field of view of a user of any of them (e.g. by being integrated into, being attached to, being under, being over, being next to, being in the foreground of, being around or being in the background of any of the following):a computer (e.g. a tablet, laptop or desktop computer), headwear (e.g. gaming headwear), eyewear (e.g. glasses or goggles), a phone (e.g. a smart phone), a projector, a visual display unit, a screen, a light or lamp (e.g. in LED form), a holographic display device, a music player (e.g. a digital music player), a tablet, a video player or video recorder, a home entertainment unit, an electronic blackboard or other viewable teaching aid, an e-reader, a cinema screen, a display unit for a vehicle (e.g. a dashboard or a windscreen display), a television, or any other (visual) display device.

The light stimulation may be arranged to be emitted from one or more of the following:
a) one or more peripheral regions of the device (e.g. from one or more regions surrounding a screen, from one or more edge or side regions of/to the screen);
b) from one or more defined areas within a central visual field;
c) from all or a substantial part of a display;
d) from a central part

The apparatus may comprise an array of multiple light-emitting entities, such as LEDs.

The invention may be viewed as including use of balanced cone excitation to control refractive error and ocular growth, thereby mitigating development of myopia. A luminaire, bulb, lamp, or any of a wide variety of components and equipment may be used to provide a light source having various wavelengths of light in a ratio and luminosity that provides a predetermined LMS cone excitation profile (or at a LS, or MS profile) that has a therapeutic effect. The light source may comprise a stand-alone light source. The light source may be characterized by a predetermined luminosity and ratio of blue light to green light to red light, wave-form, temporal frequency and amplitude of modulation .

As is explained in more detail below, it has been discovered that when the eye is stimulated with a light source with 0.2 Hz square-wave at 80% modulation, and red cones are stimulated 1.38 times more than the blue, the therapeutic effect is lost, while, for example, the therapeutic effect occurs at a ratio 0.74. It was also discovered that the therapeutic effect is also lost when the red cones are stimulated 0.05 relative to the blue. Therefore, a Red cone /Blue cone excitation ratio in a range between 1.38 and 0.05 can be used with beneficial results. However, the cone excitation ratio may be in the range 1.2 to 0.1, in the range 0.9 to 0.3, in the range 0.65 to 0.85, in the range 0.7 to 0.8, in the range 0.73 to 0.75, or may be substantially 0.74.

The invention may include one or more features disclosed in the description and/or as shown in the drawings, either individually or in combination.

### BRIEF DESCRIPTION OF THE DRAWINGS

Various embodiments of the invention will now be described by way of example only in which:
**Figure 1** is a schematic view of a light stimulation apparatus,
**Figure 2** is a flow diagram which shows the process which is implemented by the apparatus of Figure 1,
**Figure 3** shows graphs giving the relative levels of cone excitation in four different modes of light stimulation,
**Figure 4** shows various graphs relating to the results obtained from applying the different modes of light stimulation,
**Figure 5** depicts a diagram showing a display device including a region providing light stimulation to a user, and
**Figure 6** depicts a diagram showing a stand-alone display device providing light stimulation to a user.

### DETAILED DESCRIPTION

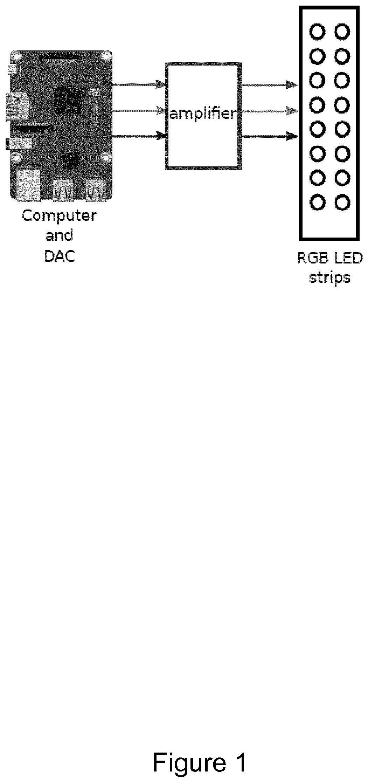

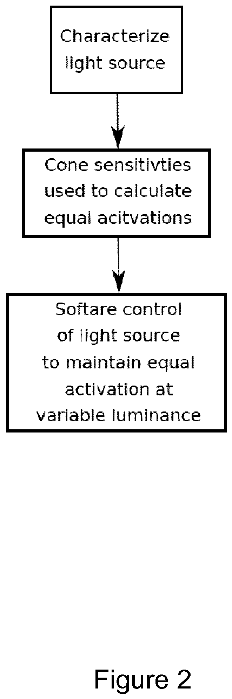

Figure 1 illustrates an apparatus for implementing the process shown in Figure 2. The apparatus and process were used experimentally with White Leghorn chicks (Gallus Gallus Domesticus, Cornell K strain; Cornell University, Ithaca, NY, USA) as test subjects. Chick cone excitations were measured for commercially available artificial illuminants and then the light sources were simulated in a controlled environment with RGB (red, green, blue) LED (light emitting diode) strips. The chick was used as a model animal system because it is a fundamental model organism for myopia development and ocular disease and it has excellent optics, color vision, and a spatial and temporal frequency sensitivity that is comparable to humans (Schaeffel & Feldkaemper 2015; Wisely 2017). For the experiment that will be described below, forty-nine, mixed sex, 10-day-old white leghorn chicks were randomly selected. After hatching, the chicks were raised in 12-12 light cycles in brooders with 300 lux fluorescent tubes (Philips LED tube 8.5T8/24 3500 K). Use of animals in this study was in compliance with the ARVO statement for the Use of Animals in Ophthalmic and Vision Research and was approved by the NECO Institutional Animal Care and Use Committee.

The chicks were placed in a 29-inch x 16-inch wire cage illuminated by the LED strips (12V LED Tape Light w/ LC4 Connector - 244 Lumens/ft.). Thirty-two, 0.5m, RGB LED strips were mounted 16 inches above the cage and controlled by the Digital to Analog Converter (DAC) of a Raspberry Pi with custom software. The LED strips were used to simulate the following lighting conditions with different ratios of short- (S), middle- (M) and long-wavelength (L) cone excitation: General Electric (GE) LED "Soft" (low S, high L), GE LED "Daylight" (medium S, high L), "Equal" (equal S, M, and L) and "High S"(high S, low L) condition. The illuminance of the light conditions was modulated in a square wave pattern at 0.2Hz though a steady light and modulation up to 5 Hz may be used. The average illuminance in the two conditions was matched. The maximum illuminance was 1790 lux and the minimum was 180 lux (average luminance of 985 lux). The Michelson contrast of the square-wave was 80% but may be as high as 100% and as low as 60%.

The cone excitation values of the two common, commercially-available light bulbs were calculated in the following steps. First, the light bulbs were positioned 16 inches above the cage floor. A lux meter (Dr. Meter LX1010BS) was used to measure the illuminance, and a spectroradiometer (Photo-Research PR-670) was used to determine the radiant power at each wavelength from 380 to 704 nm. Next, the output values of the spectroradiometer were used to calculate the L-, M-, S-, UV-, and Double- cone excitation values. Cone excitation was calculated from spectral sensitivity curves of the chick derived by Lind and Kelber (2011).

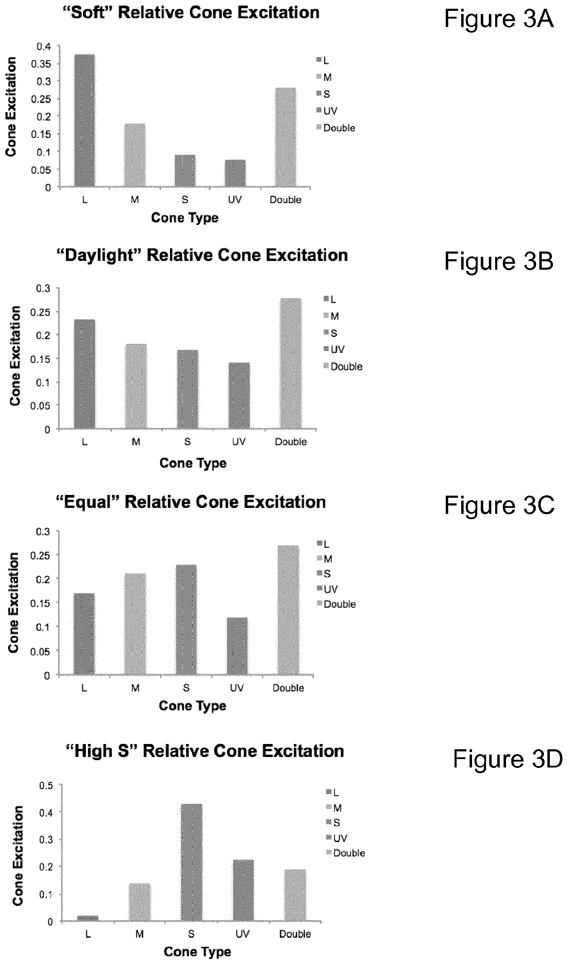

The "Equal" condition was created with relatively equal RGB components of the LED to stimulate L-, M-, and S- cone excitation with an L/S cone excitation ratio of 0.74. Figures 3A, 3B, 3C, 3D and Table 1 show the differences in the L-, M-, S-, UV-, and Double-cone excitations produced by the illuminance conditions in chick eyes.

**Table 1. The relative L-, M-, S-, UV-, and Double- cone excitation values produced by four light conditions in chick eyes compared to the total cone excitation.**

| Condition | L-cone | M-cone | S- cone | UV-cone | Double-cone | L/S ratio |
|---|---|---|---|---|---|---|
| "Soft" | 0.374 | 0.178 | 0.091 | 0.077 | 0.280 | 4.13 |
| "Daylight" | 0.232 | 0.181 | 0.168 | 0.141 | 0.278 | 1.38 |
| "Equal" | 0.170 | 0.212 | 0.229 | 0.119 | 0.270 | 0.74 |
| "High S" | 0.019 | 0.138 | 0.429 | 0.225 | 0.188 | 0.05 |

Using the calculated cone excitations from the light bulbs, the desired ratios of red, green, and blue light from the LED strips were calculated to mimic that of the light sources. These ratios were then used as the input for the LED strips.

### Measurements

Refraction and biometry were performed pre- and post-treatment. During measurement, the chicks were anesthetized with 1.5% isofluorane in oxygen (flow rate of 2 L/min). Refractions were performed with an automated infrared photoretinoscope (Schaeffel et al., 1987) and ocular components with an ocular biometer (Lenstar LS 900).

### Procedure

Chicks were 10 days old at the start of the experiment. The cage was illuminated with the modulated light source and both eyes of the chicks were exposed to their randomly assigned illuminance condition for 3 days (Tuesday noon- Friday noon). The four illuminance conditions used were "Soft" (n=13), "Daylight" (n=12), "Equal" (n=24) and "High S" (n=10).

### Analysis

The effects of the illumination conditions on the ocular components and refraction were compared based on the change between the pre- and post- condition measurements. The change in each component over the duration of the experiment with different lighting conditions was compared using Student's t- tests.

### Results

### The Effects of Indoor Illuminants on Refractive Error and Eye Growth

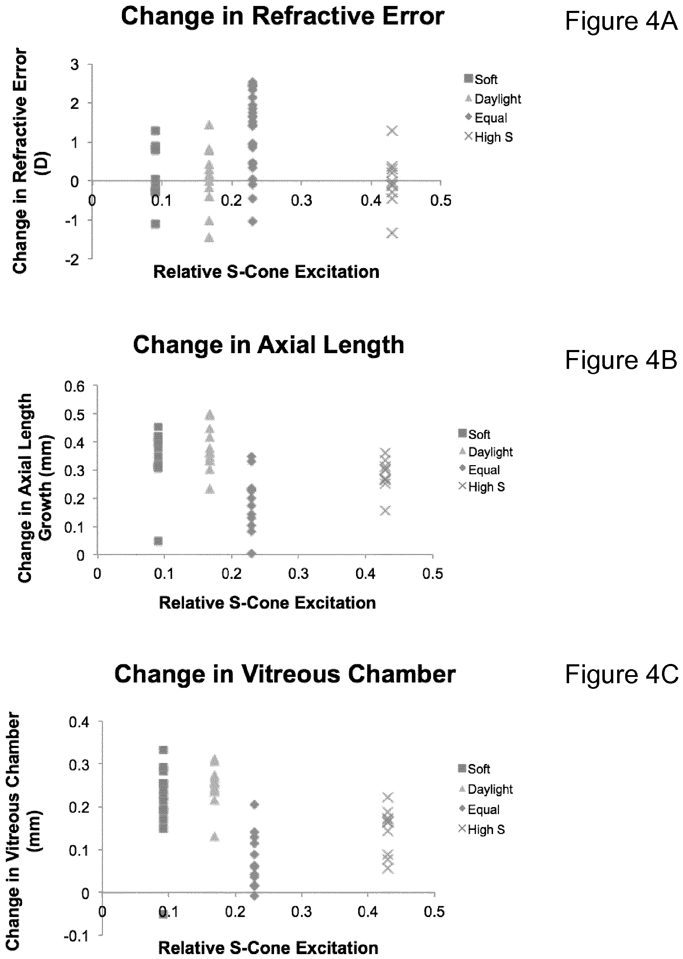

Data are shown in Figures 3A, 3B, 3C, 3D, 4A, 4B, 4C, and Table 2.

The common commercially-available light bulbs stimulated myopic eye growth, but there was no statistically significant difference in the effects that the two conditions ("Daylight" and "Soft") had on refractive error (P= 0.915; Student's t-test) and the growth of the following ocular components: axial length (P= 0.368; Student's t-test), choroid (P= 0.150; Student's t-test), vitreous (P= 0.158; Student's t-test), and anterior chamber (P= 0.7011; Student's t-test).

The mean change in refractive error in the chick eyes exposed to the "Soft" condition was +0.171 ± 0.23 D and to the "Daylight" condition was +0.088 ± 0.23D. The mean growth in axial length in the chick eyes was 0.339 ± 0.028 mm and 0.377 ± 0.023mm for the "Soft" and "Daylight" conditions, respectively. The mean growth in choroid thickness in the chick eyes was 0.011 ± 0.007 mm and 0.025 ± 0.008 mm for the "Soft" and "Daylight" conditions, respectively. The mean growth in vitreous depth in the chick eyes was 0.211 ± 0.026 mm and 0.251 ± 0.013 mm for the "Soft" and "Daylight" conditions, respectively.

### Comparison of "Soft", "Daylight", "Equal" and "High S" Conditions

The "Equal" condition showed a greater refractive error shift towards hyperopia (+1.24 ± 0.21D) compared to "Soft" (+0.17± 0.23D, Cohen's d= 1.47, t= 3.37, and p< 0.001) and "Daylight" (+0.08 ± 0.23D, Cohen's d= 1.30, t= 3.41, p < 0.001).

Birds exposed to the "Equal" condition showed a reduction in axial length growth (0.183±0.029 mm) compared to "Soft" (0.340±0.028 mm, Cohen's d= 1.44, t= 3.44, p < 0.001) and "Daylight" (0.377±0.023 mm, Cohen's d= 2.01, t= 4.69, p < 0.001). Vitreous chamber depths were also reduced (both p < 0.001).

Birds exposed to the "High S" condition experienced a 54% increase in axial length growth (0.282 ±0.018 mm) and a 92% increase in vitreous growth (0.144 ± 0.017 mm) compared to the "Equal" condition though refraction did not change (-0.02 ± 0.21 D).

**Table 2. Comparison of the mean changes in refractive error (D) and ocular component growth (mm)**

| | "Soft" | "Daylight" | "Equal" | "High S" |
|---|---|---|---|---|
| Refractive Error (D) | 0.17 ± 0.23 | 0.09 ± 0.23 | 1.24 ± 0.21 | -0.02 ± 0.21 |
| Axial Length (mm) | 0.340 ± 0.028 | 0.377 ± 0.023 | 0.183 ± 0.029 | 0.282 ± 0.018 |
| Vitreous Chamber (mm) | 0.211 ± 0.026 | 0.251 ± 0.014 | 0.075 ± 0.018 | 0.144 ± 0.017 |
| Anterior Chamber (mm) | 0.048 ± 0.022 | 0.070 ± 0.025 | -0.051 ± 0.026 | 0.036 ± 0.019 |

### Discussion

In the study reported above, we have shown that changes in refraction and eye growth are dependent on the relative cone excitation of the L- and S-cones. A ratio of L/S cone excitation of 0.74 slowed eye growth and produced less myopia than ratios of 1.38 and higher ("Soft" and "Daylight") and a ratio of 0.05 ("High S"). The L/M cone ratio had no effect on eye growth or refraction. While the spectra of common commercially-available light bulbs stimulated increased eye growth, the novel "Equal" lighting condition stimulated a 50% reduction in eye growth, which accounted for the 1.24 D hyperopic refractive changes. Reduced eye growth and hyperopic refractive changes during development reduce the likelihood of the chick developing myopia. The increased intensity of blue light in the "Equal" lighting condition enables the eye to detect the shorter focal length of blue light which promotes a slowing of eye growth and less myopia development.

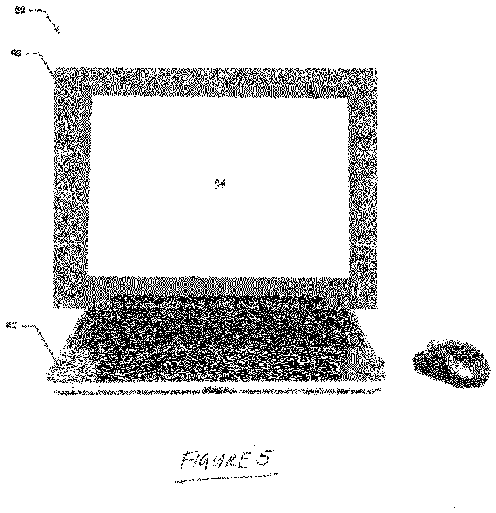

Referring now to Figure 5, one embodiment of the present invention is shown which comprises a computer 50 having a display 54 having high temporal frequencies of stimulation in the periphery 56 of the computer screen. This can be accomplished for example by having a peripheral image of a rapidly changing pattern that provides temporal stimulation. The temporal stimulation may be produced throughout the computer screen but this may not be tolerable for all users.

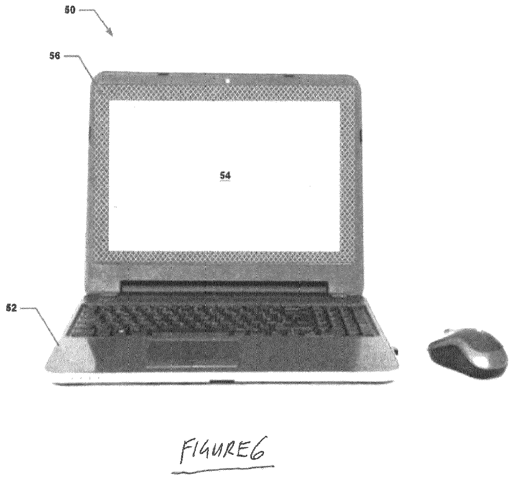

With reference to Figure 6, a further embodiment is shown. Computer 62 has a display 64. An image 66 is provided behind the display positioned to extend beyond the periphery of the computer display (or reading material) and provides the temporal stimulation. The light stimulation which is emitted has a beneficial cone excitation ratio of R/B. This is brought about by a controller which controls various characteristics of the light stimulation such as proportion of red, green and blue light emitted, a frequency with which it is emitted and the amplitude and intensity of the light stimulation emission.

In some embodiments of the invention the controller may be arranged to implement one of several preset cone excitation ratios, stored in a memory. This allows for the apparatus to be set at or close to an individual's cone excitation ratio. In some embodiments, additionally or alternatively, an operative may be able to input a specific cone excitation ratio, which has been determined as specific to a particular user, and light stimulation is then emitted at that ratio.

### References

Deng, L., Gwiazda, J., & Thorn, F. (2010). Children's refractions and visual activities in the school year and summer. Optometry and Vision Science, 87(6), 406.
Dirani, M., Tong, L., Gazzard, G., Zhang, X., Chia, A., Young, T. L., & Saw, S. M. (2009). Outdoor activity and myopia in Singapore teenage children. British Journal of Ophthalmology, 93(8), 997-1000.
Foulds, W. S., Barathi, V. A., & Luu, C. D. (2013). Progressive myopia or hyperopia can be induced in chicks and reversed by manipulation of the chromaticity of ambient light. Investigative ophthalmology & visual science, 54(13), 8004-8012.
Gottlieb, M.D. and J. Wallman, Retinal activity modulates eye growth: Evidence from rearing in stroboscopic illumination. Society of Neuroscience Abstracts, 1987. 13: p. 1297.
Guggenheim, J. A., Northstone, K., McMahon, G., Ness, A. R., Deere, K., Mattocks, C., Williams, C. (2012). Time outdoors and physical activity as predictors of incident myopia in childhood: a prospective cohort study. Investigative Ophthalmology & Visual Science, 53(6), 2856-2865.
Jones, L. A., Sinnott, L. T., Mutti, D.O., Mitchell, G. L., Moeschberger, M. L., & Zadnik, K. (2007). Parental history of myopia, sports and outdoor activities, and future myopia. Investigative ophthalmology & visual science, 48(8), 3524-3532.
Lind, O., & Kelber, A. (2011). The spatial tuning of achromatic and chromatic vision in budgerigars. Journal of Vision, 11(7), 2-2.
Mutti, D. O., Mitchell, G. L., Jones, L. A., Friedman, N. E., Frane, S. L., Lin, W. K. & Zadnik, K. (2005). Axial growth and changes in lenticular and corneal power during emmetropization in infants. Investigative ophthalmology & visual science, 46(9), 3074-3080.
Onal, S., Toker, E., Akingol, Z., Arslan, G., Ertan, S., Turan, C., & Kaplan, O. (2007). Refractive errors of medical students in Turkey: one year follow-up of refraction and biometry. Optometry And Vision Science: Official Publication Of The American Academy Of Optometry, 84(3), 175-180.
Rose, K. A., Morgan, I. G., Ip, J., Kifley, A., Huynh, S., Smith, W., & Mitchell, P. (2008). Outdoor activity reduces the prevalence of myopia in children. Ophthalmology, 115(8), 1279-1285. https://doi.org/10.1016/j.ophtha.2007.12.019
Rucker, F. J., & Wallman, J. (2008). Cone signals for spectacle-lens compensation: differential responses to short and long wavelengths. Vision Research, 48(19), 1980-1991.
Rucker, F. J., & Wallman, J. (2009). Chick eyes compensate for chromatic simulations of hyperopic and myopic defocus: evidence that the eye uses longitudinal chromatic aberration to guide eye-growth. Vision research, 49(14), 1775-1783.
Rucker, F. J., & Wallman, J. (2012). Chicks use changes in luminance and chromatic contrast as indicators of the sign of defocus. Journal of vision, 12(6), 23-23.
Rucker, F. J. (2013). The role of luminance and chromatic cues in emmetropisation. Ophthalmic and Physiological Optics, 33(3), 196-214.
Rucker, F., Britton, S., Spatcher, M., & Hanowsky, S. (2015). Blue Light Protects Against Temporal Frequency Sensitive Refractive Changes. Investigative Ophthalmology & Visual Science, 56(10), 6121-6131.
Schaeffel, F., & Feldkaemper, M. (2015). Animal models in myopia research. Clinical and Experimental Optometry, 98(6), 507-517.
Wisely, C. E., Sayed, J. A., Tamez, H., Zelinka, C., Abdel-Rahman, M. H., Fischer, A. J., & Cebulla, C. M. (2017). The chick eye in vision research: An excellent model for the study of ocular disease. Progress in retinal and eye research.

## Claims

1. Apparatus to provide light stimulation to photoreceptors of an eye of a user in a manner that is suitable for slowing myopia development in an indoor environment wherein the apparatus is arranged to emit light stimulation having a red, R, cone /blue, B, cone excitation ratio in a range of 1.38 and 0.05.

2. Apparatus as claimed in claim 1 which is arranged to output light stimulation having an R/B light excitation cone in any of the ranges 1.2 to 0.1, 0.9 to 0.3, 0.65 to 0.85, 0.7 to 0.8 or 0.73 to 0.75.

3. Apparatus as claimed in claim 2 in which the cone excitation ratio is substantially 0.74.

4. Apparatus as claimed in claim 1 or claim 2 which comprises a visual display device, or is arranged to be used in conjunction with a visual display device.

5. Apparatus as claimed in claim 3 which comprises a screen for displaying information or images to a user and said light stimulation is emitted from one or more regions of the device that are located so that they do not substantially interfere with the display of said information or images.

6. Apparatus as claimed in any preceding claim which comprises:
a display having a front surface; and
a region provided along at least one side of said front service of said display, said region providing the light stimulation to a user viewing the display.

7. Apparatus as claimed in any preceding claim which is arranged to emit a temporal light stimulation

8. Apparatus as claimed in claim 6 in which a frequency of light stimulation may be in the range 0.2Hz to 5Hz.

9. Apparatus as claimed in any preceding claim which comprises a memory which stores multiple predetermined cone excitation ratios, and the apparatus comprises a user interface which allows a user to select one of the stored ratios.

10. Apparatus as claimed in any preceding claim which comprises a controller and a user interface, and the controller arranged to receive an input from the user interface which is indicative of a specific cone excitation ratio entered by the user.

11. Apparatus as claimed in any preceding claim in which the intensity of emitted blue light of the light stimulation is at least 200 lux.

12. Apparatus as claimed in any preceding claim in which the intensity of emitted blue light of the light stimulation is at least 600 lux.
